# EUROPEAN PATENT APPLICATION

(11) **EP 4 215 540 A1**
(43) Date of publication of application: **26.07.2023**
(21) Application number: 21869571.6
(22) Date of filing: 23.08.2021
(51) Int. Cl.: C07J 41/00, A61K 31/575

(54) **METHOD FOR MASS-PRODUCING SODIUM TAURODEOXYCHOLATE**

(30) Priority: 18.09.2020 US 202063080151 P
(71) Applicant: Seoul National University R & DB Foundation, Seoul 08826 (KR); SHAPERON Inc., Seoul 06373 (KR)
(72) Inventor: SEONG, Seung Yong, Seoul 05507 (KR)
(74) Representative: Vossius & Partner Patentanwälte Rechtsanwälte mbB
(86) International application number: PCT/KR2021/011234
(87) International publication number: WO 2022/059948

(57) **Abstract**

The present invention relates to a mass production method of sodium taurodeoxycholate having low impurity and few by-products, in which the procedure of process is simplified since isopropyl alcohol is used and separation and purification is performed through batch washing for commercial production of sodium taurodeoxycholate.

## Description

### [Technical Field]

The present invention relates to a mass production method of sodium taurodeoxycholate, more particularly to a mass production method of sodium taurodeoxycholate, comprising 1) a step of synthesizing crude sodium taurodeoxycholate; 2) a step of washing the crude sodium taurodeoxycholate synthesized in step 1) using an organic solvent and performing filtration to obtain a cake; and 3) a purification step of mixing the cake obtained in step 2) with a solution containing isopropyl alcohol, then i) stirring the mixture with heating for dissolution, ii) stirring the solution with cooling for recrystallization, and iii) washing the recrystallized sodium taurodeoxycholate with isopropyl alcohol and performing filtration.

### [Background Art]

Bile acids or bile salts are substances secreted by the liver and gallbladder, include glycocholic acid, taurocholic acid, and the like, and are substances related to the mechanism of action of cholesterol and the like. There are many kinds of bile acids, including cholic acid, chenodeoxycholic acid, taurocholic acid, lithocholic acid and the like, and each of these substances has been continuously investigated as being useful for the treatment and prevention of diseases.

Taurodeoxycholic acid, a kind of bile acid, corresponds to almost the only form of sulfonic acid that exists in nature so far. It has been known that sodium taurodeoxycholate, which is a salt form of taurodeoxycholic acid, reduces blood IgE content as a GPCR19 agonist, reduces TH2 cytokine levels, increases the TH1 cytokine level, and can be used as a composition for prevention or treatment of atopy (Korean Patent No. 10-1998402), and sodium taurodeoxycholate improves cognitive and behavioral disorders, inhibits brain tissue apoptosis, enhances immunity, and reduces the formation of amyloid beta plugs to exhibit an inhibitory or therapeutic effect on Alzheimer's disease and dementia (Korean Patent No. 10-1743960).

However, in order for such sodium taurodeoxycholate to be actually developed as a drug, it is required to be possible to mass-produce sodium taurodeoxycholate, but in the mass production of sodium taurodeoxycholate, there are problems such as various by-products that are inevitably generated during the synthesis process, unreacted starting materials, and the fact that there is no significant difference in water solubility and solubility, and there have been thus difficulties in effective separation and purification because of these problems. In the case of performing purification according to a generally known method, there are difficulties in repeated commercial production because of recovery problems, reuse problems, yield problems, and complicated separation process.

In this regard, Korean Patent No. 10-0396113 discloses a method for manufacturing taurodeoxycholic acid by bilayer extraction using an organic solvent and an acidic or alkaline aqueous solution as a method for purifying taurodeoxycholic acid, and Korean Patent No. 10-2068381 discloses a method for manufacturing a bile acid derivative using a bile acid together with RuCl₃, NaIO₄, and an acid. However, there is no known method for synthesizing sodium taurodeoxycholate, there is no known method for purifying taurodeoxycholic acid, and there is no known mass production method to a commercially repeatable extent.

Accordingly, the present inventors have completed the present invention by newly finding out that the procedure of process is simple, the purity is high, and mass production is possible in the case of performing separation and purification through batch washing using isopropyl alcohol as a mass production method of sodium taurodeoxycholate.

### [Summary of Invention]

### [Technical Problem]

An object of the present invention is to provide a mass production method of sodium taurodeoxycholate having high purity and few by-products, in which the procedure of process is simplified since separation and purification is performed through batch washing using isopropyl alcohol, in order to solve the problem that it is difficult to mass-produce sodium taurodeoxycholate and the process is complicated in the prior art.

### [Solution to Problem]

In order to solve the problems, the present invention provides a mass production method of sodium taurodeoxycholate, comprising 1) a step of synthesizing crude sodium taurodeoxycholate; 2) a step of washing the crude sodium taurodeoxycholate synthesized in step 1) using an organic solvent and performing filtration to obtain a cake; and 3) a purification step of mixing the cake obtained in step 2) with a solution containing isopropyl alcohol, then i) stirring the mixture with heating for dissolution, ii) stirring the solution with cooling for recrystallization, and iii) washing the recrystallized product with isopropyl alcohol and performing filtration.

The present invention also provides sodium taurodeoxycholate manufactured by the mass production method.

By the mass production method of sodium taurodeoxycholate of the present invention, it is possible to manufacture 1 kg or more at one time of production.

In an aspect of the present invention, the crude sodium taurodeoxycholate in step 1) is synthesized by placing a solution containing sodium taurate, deoxycholic acid, and N-ethoxycarbonyl-2-ethoxy-1,2-dihydroquinoline (EEDQ) in a stirrer and stirring the solution while adjusting the temperature.

In an aspect of the present invention, the organic solvent in step 2) is one or more selected from the group consisting of ethanol, acetone, pyridine, hexafluoroisopropanol, propanol, butanol, cyclohexane, toluene, dichloromethane, diethyl ether, ethyl acetate, methyl acetate, and a mixed solvent of two or more thereof.

In a specific aspect of the present invention, the organic solvent in step 2) is a mixed solvent containing ethanol and acetone, in which a mixed volume ratio of ethanol to acetone is 1:0.5 to 2.

In an aspect of the present invention, step 3) is repeated two or more times. In a specific aspect of the present invention, step 3) is repeated two to three times.

In an aspect of the present invention, the solution containing isopropyl alcohol in step 3) contains water and isopropyl alcohol, in which a mixed volume ratio of water to isopropyl alcohol is 1:1 to 10.

In an aspect of the present invention, the heating in i) of step 3) is heating at 20°C to 100°C.

In an aspect of the present invention, the cooling in ii) of step 3) is cooling at 0°C to 50°C.

In an aspect of the present invention, the stirring in ii) of step 3) is stirring for 8 to 30 hours.

In an aspect of the present invention, isopropyl alcohol in the solution containing isopropyl alcohol in step 3) is 5 to 20 times the weight of the cake in step 2) .

In an aspect of the present invention, the mass production method of sodium taurodeoxycholate may further comprise 4) a step of purifying sodium taurodeoxycholate obtained in step 3) using a mixed solution containing acetone.

In a specific aspect of the present invention, step 4) may include A) a step of dissolving sodium taurodeoxycholate filtered in step 3) in a mixed solution containing acetone; B) a step of adding acetone dropwise to the solution prepared in step A), and recrystallizing sodium taurodeoxycholate while performing cooling and stirring; and C) a step of washing the recrystallized sodium taurodeoxycholate with acetone and performing filtration and drying.

### [Advantageous Effects of Invention]

The present invention relates to a mass production method of sodium taurodeoxycholate, and has an advantage that the industrial production of sodium taurodeoxycholate is possible since the procedure of process is simplified, mass production is possible, and the purity is high by performing purification using isopropyl alcohol and batch washing.

### [Brief Description of Drawings]

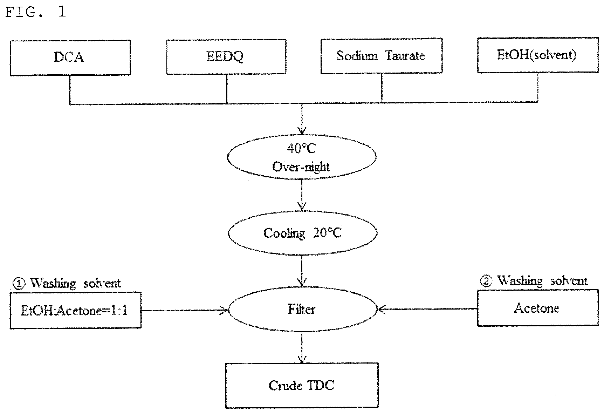

FIG. 1 is a schematic diagram of a mass production method of the present invention;

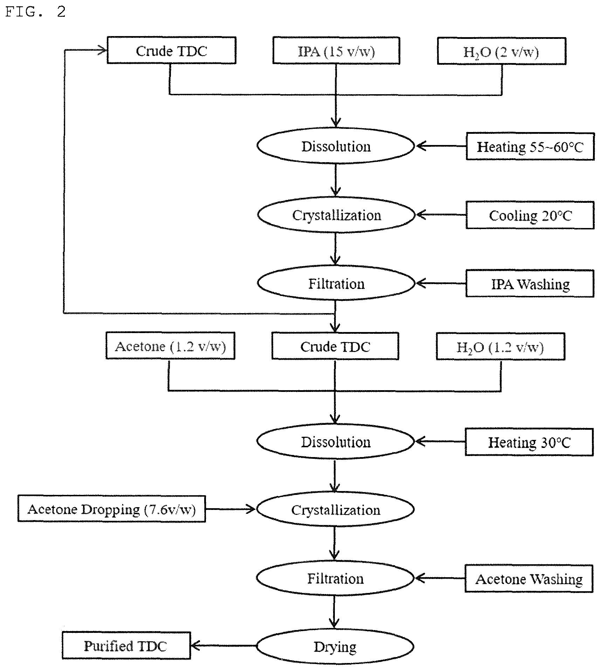

FIG. 2 is a diagram illustrating a purification process in a mass production method of the present invention;
FIG. 3 is a diagram illustrating a scheme of a mass production method of the present invention;
FIG. 4 is a diagram confirming whether sodium taurodeoxycholate manufactured according to a production method of the present invention is synthesized through chromatography; and
FIG. 5 is a diagram confirming the purity of sodium taurodeoxycholate manufactured according to a production method of the present invention through HPLC.

### [Description of Embodiments]

Sodium taurodeoxycholate is mass-produced using a solution containing isopropyl alcohol in the manufacture thereof.

### [Embodiments]

Hereinafter, the present invention will be described in detail.

Throughout the specification of the present invention, when a part "includes" a certain component, this means that the part does not exclude other components but may further include other components unless otherwise stated.

Compounds refer to commonly recognized compounds unless otherwise defined herein.

The present invention relates to a mass production method of sodium taurodeoxycholate, comprising 1) a step of synthesizing crude sodium taurodeoxycholate; 2) a step of washing the crude sodium taurodeoxycholate synthesized in step 1) using an organic solvent and performing filtration to obtain a cake; and 3) a purification step of mixing the cake obtained in step 2) with a solution containing isopropyl alcohol, then i) stirring the mixture with heating for dissolution, ii) stirring the solution with cooling for recrystallization, and iii) washing the recrystallized product with isopropyl alcohol and performing filtration.

The present invention also relates to sodium taurodeoxycholate manufactured by the mass production method.

In the present invention, as the reactants for manufacturing crude sodium taurodeoxycholate, for example, unprocessed deoxycholic acid, taurine, sodium taurate, taurodeoxycholic acid derivatives, and the like may be used. As the reactants, commercially available ones may be used, or those synthesized by methods known in the art or those obtained by collecting from nature and then being subjected to treatment may be used. These are illustrative, and the reactants are not limited to the substances or methods described above.

Specifically, in an aspect of the present invention, the crude sodium taurodeoxycholate in step 1) may be synthesized by placing a solution containing sodium taurate, deoxycholic acid, and N-ethoxycarbonyl-2-ethoxy-1,2-dihydroquinoline (EEDQ) in a stirrer and stirring the solution while adjusting the temperature.

More specifically, synthesized sodium taurate and deoxycholic acid may be used, sodium taurate may be manufactured by mixing taurine with a sodium salt, and deoxycholic acid may be obtained by crystallizing natural deoxycholic acid, but the reactants not limited thereto, and include all those manufactured by known methods or procured by commercial methods. In the present invention, the reactants may be used in suitable amounts for mass production of crude sodium taurodeoxycholate.

In the present invention, EEDQ is a compound represented by the following Chemical Formula, and may be expressed as IUPAC names 2-ethoxy-2H-quinoline-1-carboxylic acid ethyl ester and ethyl 1,2-dihydro-2-ethoxyquinoline-1-carboxylate.

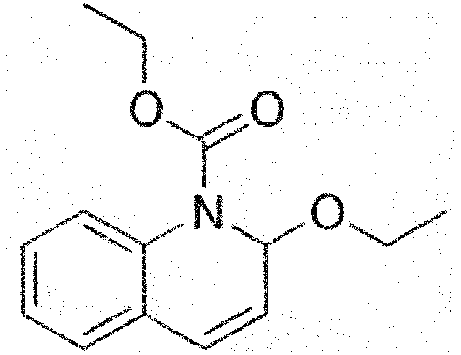

Specifically, in the present invention, when sodium taurate, deoxycholic acid and EEDQ are used, synthesis may proceed by the following chemical reactions.

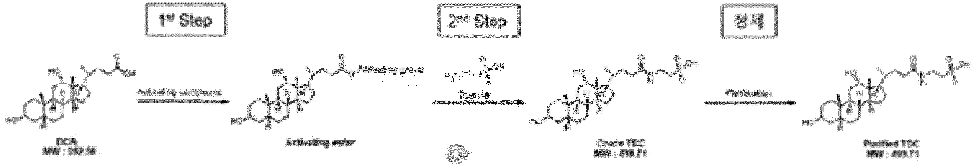

In the present invention, step 1) may be to synthesize sodium taurodeoxycholic acid by synthesizing the reactants. According to a specific synthesis method according to an aspect of the present invention, deoxycholic acid and EEDQ (N-ethoxycarbonyl-2-ethoxy-1,2-dihydroquinoline) may react to form an intermediate together with quinoline as a by-product, and the intermediate and sodium taurate may react to form sodium taurodeoxycholate. At this time, the solvent may be ethanol.

More specifically, in the present invention, by using sodium taurate, deoxycholic acid and EEDQ, high reactivity may be acquired, decreased side reactions take place, and the stability during the reaction is high at 40°C in the first step at the time of mass synthesis. There are advantages that high reactivity, decreased side reactions, and stability during reaction are maintained in the second step, and moreover, sodium taurodeoxycholate is obtained as a solid after the reaction, and the free active group can be removed using a solvent, so that the free active group is easily removed. Through the purification, sodium taurodeoxycholate containing single impurities at less than 0.1% may be obtained with high yield and high purity.

The amount of the reactants, temperature and stirring in step 1) may be adjusted to proper levels depending on the amount of sodium taurodeoxycholate to be synthesized. The proper levels may be determined by analyzing the substance produced in step 1), unreacted substances, and the like.

Specifically, although not limited thereto, step 1) may include a step of stirring with heating and a step of stirring with cooling to synthesize sodium taurodeoxycholate.

In the synthesis step, the temperature during heating may be 30°C to 100°C, more specifically, stirring may be performed while performing heating at 30°C to 80°C, 31°C to 70°C, 32°C to 60°C, 33°C to 55°C, 34°C to 50°C, or 35°C to 45°C. However, it may be undesirable that the heating temperature exceeds 100°C in terms of time required for purification and economic feasibility since the amount of impurities produced remarkably increases. When the heating temperature corresponds to about 40°C, decreased side reactions take place and high stability is exhibited.

In the synthesis step, the step of stirring with heating may be stirring for 10 to 30 hours, more specifically for 12 to 28 hours or 14 to 26 hours. Specifically, crystals may be formed after 0.5 to 5 hours from the start of stirring, and a step of releasing the generated crystals so that the crystals do not agglomerate and a high-yield synthesis occurs may be further included.

In the synthesis step, the temperature during cooling may be 0°C to 30°C, more specifically, 10°C to 30°C or 15°C to 25°C. The temperature is required to be maintained at 0°C or higher since the impurity increases when the temperature decreases to below 0°C during cooling, and it is favorable for crystallization when the temperature during cooling is about 20°C.

In the synthesis step, the step of stirring with cooling may be stirring for 0.1 to 5 hours, more specifically, the stirring time may be 0.2 to 3 hours, 0.3 to 2 hours, 0.4 to 2 hours, or 0.5 to 2 hours.

In the present invention, step 2) relates to a step of washing and filtering the substance produced in step 1) .

In an aspect of the present invention, the organic solvent in step 2) is one or more selected from the group consisting of ethanol, acetone, pyridine, hexafluoroisopropanol, propanol, butanol, cyclohexane, toluene, dichloromethane, diethyl ether, ethyl acetate, methyl acetate, and a mixed solvent of two or more thereof. The organic solvent is one or more kinds selected, and used as a single solvent, or includes a mixed solvent in which two or more kinds are mixed.

In a specific aspect of the present invention, the organic solvent in step 2) is a mixed solvent containing ethanol and acetone, in which the mixed volume ratio of ethanol to acetone is 1:0.5 to 2. More specifically, the organic solvent may be a mixed solvent having a mixed volume ratio of ethanol to acetone of 1:0.6 to 1.4, 0.75 to 1.25, or 0.8 to 1.2.

In the present invention, step 3) relates to a step of purifying the substance obtained in steps 1) and 2). In the present invention, step 3) is to perform purification through batch washing, and thus purification is performed without the process of drying the substance obtained in steps 1) and 2). The batch washing is not a conventional column method, thus the procedure of process is simplified, the impurity is low, the process may be performed on a large scale, and sodium taurodeoxycholate may be produced on a large scale.

In an aspect of the present invention, step 3) is repeated two or more times. In a specific aspect of the present invention, step 3) may be repeated two to three times. The number of repetition of step 3) may be selected in consideration of purity and yield. Purification may be performed by a simple process since the process of drying is not performed between the respective numbers of repetitions.

The amount of isopropyl alcohol used, temperature, stirring time, and the like in step 3) may be adjusted to proper levels depending on the amount of sodium taurodeoxycholate to be synthesized.

In a specific aspect of the present invention, the solution containing isopropyl alcohol in step 3) may contain water and isopropyl alcohol, and the mixed volume ratio of water to isopropyl alcohol may be 1:1 to 10. In a more specific aspect of the present invention, the mixed volume ratio may be specifically 1:5 to 10 or 1:6 to 9.

In a specific aspect of the present invention, the heating in i) of step 3) is heating at 20°C to 100°C. Specifically, the heating may be heating at 30°C to 90°C, 35°C to 85°C, 40°C to 80°C, 45°C to 75°C, 46°C to 74°C, 47°C to 73°C, 48°C to 72°C, 49°C to 71°C, or 50°C to 70°C followed by stirring. The heating in i) of step 3) may be to completely dissolve the substance in a mixed solution containing isopropyl alcohol and stir the solution, and may be to further perform stirring for 10 to 30 minutes after confirmation of the dissolution of the substance. The reaction is excellent at about 60°C in terms of purification time and stability, although not limited thereto.

In a specific aspect of the present invention, the cooling in ii) of step 3) is cooling at 0°C to 50°C. A specific cooling temperature may be 10°C to 40°C, 15°C to 35°C, 15°C to 34°C, 15°C to 33°C, 15°C to 32°C, 15°C to 31°C, or 15°C to 30°C, cooling may be performed to form crystals, and cooling may be performed slowly since crystallization may not be performed smoothly when cooling is performed rapidly.

In a specific aspect of the present invention, the stirring in ii) of step 3) is stirring for 8 to 30 hours. A more specific stirring time may be 8 to 25 hours, 8 to 20 hours, or 9 to 15 hours. The stirring time may be adjusted to a proper level in consideration of the amount of the reactants and the temperature.

In an aspect of the present invention, isopropyl alcohol in the solution containing isopropyl alcohol in step 3) may be used in to be 5 to 20 times the weight of the cake in step 2). Specifically, the isopropyl alcohol used includes the amount of all isopropyl alcohol used in step 3), and means the weight of isopropyl alcohol excluding water in the case of being used in mixture with water. More specifically, the isopropyl alcohol may be 5 to 15 times, 6 to 14 times, or 7 to 12 times the weight of the cake, and the degree of recrystallization is the most favorable in the above range.

In an aspect of the present invention, the mass production method of sodium taurodeoxycholate may further comprise step 4) after step 3), and step 4) may be a step of purifying sodium taurodeoxycholate obtained in step 3) using a mixed solution containing acetone.

In a specific aspect of the present invention, step 4) may include A) a step of dissolving sodium taurodeoxycholate filtered in step 3) in a mixed solution containing acetone; B) a step of adding acetone dropwise to the solution prepared in step A), and recrystallizing sodium taurodeoxycholate while performing cooling and stirring; and C) a step of washing the recrystallized sodium taurodeoxycholate with acetone and performing filtration and drying.

In the present invention, step 4) may be further included, and does not correspond to the essential configuration of the present invention, and a purity of 99.5% or more may be achieved when step 4) is further included.

In a specific aspect of the present invention, the mixed solution containing acetone in step A) of step 4) contains water and acetone, and the mixed volume ratio of water to acetone is 1:1 to 10. In a more specific aspect of the present invention, the mixed volume ratio of water to acetone in the mixed solvent in step A) may be specifically 1:5 to 10 or 1:6 to 9.

In a specific aspect of the present invention, step B) of step 4) is a step of adding acetone dropwise to the solution prepared in step A) and recrystallizing sodium taurodeoxycholate while performing cooling and stirring, and includes all of a step of adding acetone dropwise to the solution prepared in step A) and then performing stirring, a step of performing stirring and then adding acetone dropwise or a step of stirring while adding acetone dropwise, as well as a step of stopping the dropwise addition of acetone and performing stirring when crystals are formed by dropwise addition of acetone and stirring, and the order of dropwise addition, cooling and stirring is not limited.

In a specific aspect of the present invention, step C) of step 4) may be carried out at 10°C to 40°C, more specifically at 15°C to 35°C, 15°C to 34°C, 15°C to 33°C, 15°C to 32°C, 15°C to 31°C, or 15°C to 30°C, and the purpose and effect of cooling are as mentioned above.

In an aspect of the present invention, acetone in step 4) may be used in a weight to be 5 to 20 times, more specifically 5 to 15 times, 5.5 to 12 times, or 6 to 12 times the weight of the cake filtered in step 3). This includes the amount of all acetone used in steps A) to C) of step 4), and means the weight of acetone excluding water in the case of being used in mixture with water.

In an aspect of the present invention, by the mass production method of sodium taurodeoxycholate, it is possible to manufacture 1 kg or more at one time of production.

In an aspect of the present invention, the yield of sodium taurodeoxycholate may be 25% or more. More specifically, the yield may be 25% or more, 26% or more, 27% or more, 280 or more, 29% or more, or 30% or more.

In an aspect of the present invention, by the mass production method of sodium taurodeoxycholate, it is possible to manufacture sodium taurodeoxycholate having a purity of 99% or more. More specifically, the purity may be 99.1% or more, 99.15% or more, 99.2% or more, 99.25% or more, 99.3% or more, 99.35% or more, 99.4% or more, 99.45% or more, or 99.5% or more.

In the present invention, by the mass production method of sodium taurodeoxycholate, it is possible to manufacture sodium taurodeoxycholate having a purity of 99% or more by 1 kg or more at one time of production with a yield of 25% or more, and thus it is possible to efficiently manufacture sodium taurodeoxycholate.

### [Examples]

Hereinafter, the present invention will be described in detail by way of Examples and Experimental Examples.

However, the following Examples and Experimental Examples are merely illustrative of the present invention, and the contents of the present invention are not limited to the following Examples and Experimental Examples.

### <Example 1> Large-scale synthesis of sodium taurate

Into a reactor, 2.04 kg of sodium hydroxide and 44 L of ethanol were charged, and stirring was started. During stirring, the external temperature was set to 55°C and the internal temperature was raised to 55°C, it was confirmed that sodium hydroxide was completely dissolved, and then 5.5 kg of taurine was charged. After charging of taurine was completed, the mixture was stirred for 2 hours or more. After the stirring was completed, the external temperature was set to 30°C, the internal temperature was lowered to 30°C, and the mixture was stirred for 1 hour. After stirring was completed, washing/filtration was performed using a Nutsche filter, and washing/filtration was performed such that washing/filtration with 27.5 L of ethanol was first performed and then washing/filtration with 16.5 L of acetone was further performed. The wet cake was placed on a tray, transferred to a dryer, and then dried in a vacuum at 35°C for 12 hours or more to obtain 5.28 kg of sodium taurate.

### <Example 2> Large-scale synthesis of sodium taurodeoxycholate

Into a reactor, 1.96 kg of sodium taurate prepared in <Example 1>, 5.5 kg of deoxycholic acid, 6.93 kg of EEDQ (**N-ethoxycarbonyl-2-ethoxy-1,2-dihydroquinoline**), and 55 L of ethanol were charged, and stirring was started. During stirring, the external temperature was set to 40°C and the internal temperature was raised to 40°C, and the mixture was stirred for 15 hours or more. After the stirring was completed, the external temperature was set to 20°C and the internal temperature was lowered to 20°C. After cooling was completed, the mixture was stirred for 1 hour. After the stirring was completed, washing/filtration was performed using a Nutsche filter, and washing/filtration was performed such that two times of washing/filtration with a total of 55 L of a mixed solution (mixed volume ratio 1:1) of ethanol and acetone were performed, and washing/filtration with 16.5 L of acetone was performed to obtain 7.515 kg of a first cake.

### <Example 3-1> Large-scale purification (1) of sodium taurodeoxycholate

Into a reactor, 7.515 kg of the first cake prepared in <Example 2>, 76.1 L of isopropyl alcohol, and 10.1 L of purified water were charged, and stirred. During stirring, the external temperature was set to 60°C and heating was performed. After complete dissolution was confirmed, the mixture was further stirred for 20 minutes. After that, cooling was slowly performed to 20°C for 3 hours and then stirring was further performed for 12 hours or more. After the stirring was completed, washing/filtration was performed using a Nutche filter. At this time, washing/filtration with 15.6 L of isopropyl alcohol was performed to obtain 13.13 kg of a second cake.

Thereafter, 13.13 kg of the second cake, 56.8 L of isopropyl alcohol, and 7.6 L of purified water were charged into a reactor, and stirred. During stirring, the external temperature was set to 60°C and heating was performed. After complete dissolution was confirmed, the mixture was further stirred for 20 minutes. Thereafter, cooling was slowly performed to 20°C for 3 hours and then stirring was performed for 12 hours or more. After the stirring was completed, washing/filtration was performed using a Nutsche filter. At this time, washing/filtration with 11.4 L of isopropyl alcohol was performed to obtain 7.88 kg of a 3rd cake.

Thereafter, 7.88 kg of 3rd cake, 42.6 L of isopropyl alcohol, and 5.7 L of purified water were charged into a reactor, and stirred. During stirring, the external temperature was set to 60°C and heating was performed. After complete dissolution was confirmed, the mixture was further stirred for 20 minutes. Thereafter, cooling was slowly performed to 20°C for 3 hours and then stirring was further performed for 12 hours or more. After the stirring was completed, washing/filtration was performed using a Nutsche filter. At this time, washing/filtration with 8.5 L of isopropyl alcohol was performed to obtain a wet solid. The wet solid was weighed, and the measured weight was 4.73 kg. The wet solid was placed on a tray, transferred to a dryer, and then dried in a vacuum at 35°C for 6 hours or more to obtain 2.84 kg of sodium taurodeoxycholate (purity: 99.0%).

### <Example 3-2> Large-scale purification (1) of sodium taurodeoxycholate

The large-scale purification of sodium taurodeoxycholate was performed through the same procedure as in Example <3-1> except that the weight of the first cake was changed. The first cake was charged by 6.91 kg to obtain 11.34 kg of the second cake, 6.81 kg of the 3rd cake, 4.08 kg of the wet solid, and finally 2.45 kg of sodium taurodeoxycholate (purity: 99.9%).

### <Example 4> Large-scale purification (2) of sodium taurodeoxycholate

Into a reactor, 2.84 kg of sodium taurodeoxycholate purified in <Example 3-1>, 2.45 kg of sodium taurodeoxycholate purified in <Example 3-2>, 64.2 L of isopropyl alcohol, and 8.6 L of purified water were charged, and stirred. During stirring, the external temperature was set to 60°C and heating was performed. After complete dissolution was confirmed, the mixture was further stirred for 20 minutes. After that, cooling was slowly performed to 20°C for 3 hours and then stirring was further performed for 12 hours or more. After the stirring was completed, washing/filtration was performed using a Nutche filter. At this time, washing/filtration with 12.8 L of isopropyl alcohol was performed to obtain 6.89 kg of wet cake.

Thereafter, 6.89 kg of the wet cake, 47.6 L of isopropyl alcohol, and 6.4 L of purified water were charged into a reactor, and stirred. During stirring, the external temperature was set to 60°C and heating was performed. After complete dissolution was confirmed, the mixture was further stirred for 20 minutes. After that, cooling was slowly performed to 20°C for 3 hours and then stirring was further performed for 12 hours or more. After the stirring was completed, washing/filtration was performed using a Nutsche filter. At this time, washing/filtration with 9.7 L of isopropyl alcohol was performed to obtain a wet solid. The wet solid was weighed, and the measured weight was 5.17 kg. The wet cake was placed on a tray, transferred to a dryer, and then dried in a vacuum at 35°C for 6 hours or more to obtain 3.10 kg of sodium taurodeoxycholate.

### <Example 5> Large-scale purification (3) of sodium taurodeoxycholate

Into a reactor, 3.10 kg of sodium taurodeoxycholate obtained in <Example 4>, 2.9 L of acetone, and 2.9 L of purified water were charged, and stirring was started. During stirring, the external temperature was set to 30°C and the internal temperature was raised to 30°C to achieve complete dissolution. Thereafter, the obtained solution was filtered through a 0.45 µm cartridge filter, and the filtrate was transferred to a crystallizer.

Thereafter, 8.3 L of acetone was added dropwise to the crystallizer, and the dropwise addition of acetone was stopped when crystals were formed, followed by stirring for 1 hour. After the stirring was completed, 10 L of acetone was added dropwise while stirring was performed. After the dropwise addition was completed, the mixture was cooled to 20°C and further stirred for 2 hours. After the stirring was completed, washing/filtration was performed using a Nutche filter. At this time, washing/filtration with 7.2 L of acetone was performed to obtain 5.92 kg of a wet cake. The wet cake was placed on a tray, transferred to a dryer, and then dried in a vacuum at 70°C for 12 hours or more to obtain 2.61 kg of crystallized sodium taurodeoxycholate (yield: 2512%, purity: 99.9% or more).

### <Example 6> Lyophilization of sodium taurodeoxycholate

A solution was prepared by dissolving 2.61 kg of crystallized sodium taurodeoxycholate obtained in <Example 5> in 25.0 L of distilled water, then placed on a tray, and transferred to a lyophilizer. Thereafter, the lyophilizer was set as shown in Table 1 below, and then lyophilization was performed. Through lyophilization, 2.57 kg of crystallized product was obtained, the crystallized product obtained through lyophilization was pulverized for 90 minutes using a pulverizer at a pulverizing net size of 0.99 mm and a rotation speed of 3000 rpm to obtain crystallized sodium taurodeoxycholate.

**[Table 1]**

| | Freeze | Primary | | | | | | | |
|---|---|---|---|---|---|---|---|---|---|
| | SV01 | SV02 | SV03 | SV04 | SV05 | SV06 | SV07 | SV08 | SV09 |
| Temperature (°C) | -40 | -20 | -20 | 0 | 0 | 20 | 20 | 35 | 35 |
| VAC. (mmTorr) | - | 10 | 10 | 10 | 10 | 10 | 10 | 10 | 10 |
| Set time (min) | 180 | 180 | 1260 | 180 | 450 | 180 | 540 | 135 | 675 |

### <Experimental Example 1> Confirmation of synthesis of sodium taurodeoxycholate

Sodium taurodeoxycholate synthesized in <Example 2> was sampled and analyzed through HPLC. HPLC was performed under the conditions shown in Table 2. The mobile phase was prepared by mixing 4.0 g of sodium dihydrogenphosphate monohydrate, 0.606 g of sodium dodecyl sulfate, 1000 ml of water, and 515 ml of acetonitrile together, and then adjusting the pH of the mixture to 2.1 using phosphoric acid. The sample was prepared by adding 50 mg of the product into a 10 ml flask, dissolving the product in 3.0 ml of methanol, filling up the flask to the mark with the mobile phase, filtering the solution through a 0.45 um membrane syringe filter, and placing the filtrate in an HPLC vial. As a result of the analysis, it was confirmed that sodium taurodeoxycholate was synthesized.

**[Table 2]**

| Item | Condition |
|---|---|
| Detector | 220 nm |
| Column | C18, 5 um, 4.6 mm(I.D) * 250 mm(length) |
| Temperature | 40°C |
| Flow rate | 1.0 ml/min |
| Injection Volume | 30 µl |
| Run time | 105 min |

### <Experimental Example 2> Confirmation of detection of unreacted substances and sodium taurodeoxycholate

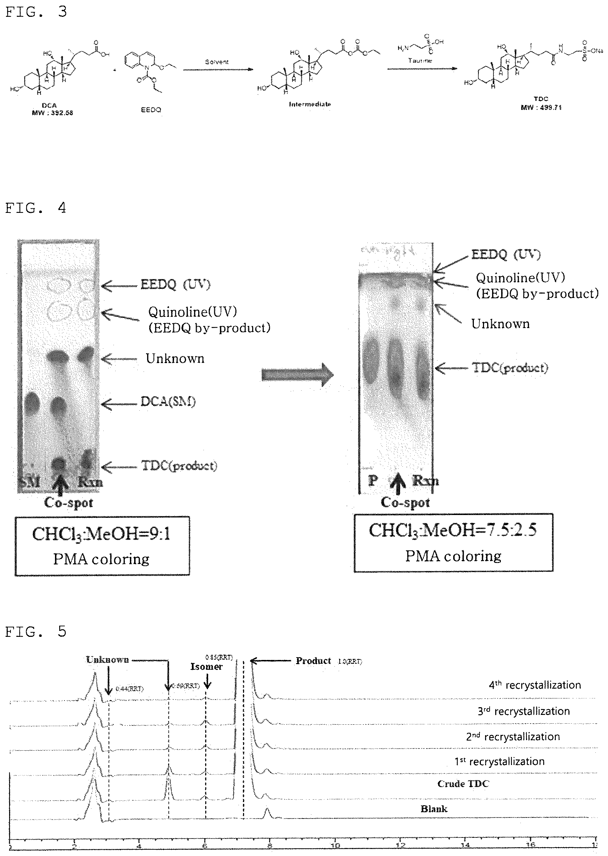

In <Example 2>, when the crystals settled after stirring, the ethanol solution of the supernatant was sampled to confirm whether the reactants were detected through TLC. The analysis was performed using 1) CHCl₃:MeOH = 9:1 and PMA coloring solvent for deoxycholic acid, 2) IPA:H₂O = 8:2 and Ninhydrine coloring solvent for taurine, and 3) CHCl₃:MeOH = 7.5:2.5 solvent for sodium taurodeoxycholate, respectively, as the developing solvent for TLC. EEDQ and quinoline, a by-product, were visually confirmed at UV 254 nm. As a result of the analysis, as illustrated in FIG. 4 , it was confirmed that deoxycholic acid, a starting material, was not present.

### <Experimental Example 3> Confirmation of purity of sodium taurodeoxycholate depending on number of purification (recrystallization)

### <Experimental Example 3-1> Impurity detecting test

In order to confirm the purity of sodium taurodeoxycholate prepared according to Examples above, detection and loss on drying of sodium deoxycholate, related substances, heavy metals, taurine, and residual solvents were confirmed.

For detection, a sample solution prepared by dissolving a certain amount of sodium taurodeoxycholate prepared according to Examples in a solvent and a standard solution prepared by dissolving a certain amount of standard sodium taurodeoxycholate in a solvent were prepared, and measurement was performed using an ultraviolet absorptiometer. Loss on drying was measured in conformity with the Korean Pharmacopoeia General Test Method Loss on Drying Test Method, and heavy metals were measured in conformity with the Korean Pharmacopoeia General Test Method Heavy Metals Test Method No. 1.

The analysis results are shown in **Tables 3 to 8 below.**

**[Table 3]**

| Item | Amount of specimen taken (mg) | Peak area | Related substances (%) | Average (%) |
|---|---|---|---|---|
| Sodium deoxycholate | 50.02 | N/D | Below detection limit | Below detection limit |
| | 50.13 | N/D | Below detection limit | |
| | 50.05 | N/D | Below detection limit | |
| - Test criteria: 1.0% or less | | | | |

**[Table 4]**

| Item | | Related substances content (%) | | |
|---|---|---|---|---|
| | Test solution 1 (400.12 mg) | Test solution 2 (400.11 mg) | Test solution 3 (400.15 mg) | Average |
| Sodium taurocholate | 0.1820 | 0.2094 | 0.1870 | 0.1928 |
| Unknown | 0.0628 | 0.0689 | 0.0628 | 0.0648 |
| Sodium taurochenodeoxycholate | 0.3374 | 0.3417 | 0.3340 | 0.3377 |
| Sodium taurodeoxycholate | 99.4090 | 99.3680 | 99.4060 | - |
| Unknown | 0.0023 | 0.0022 | 0.0027 | 0.0024 |
| Unknown | 0.0000 | 0.0000 | 0.0020 | 0.0006 |
| Unknown | 0.0038 | 0.0037 | 0.0033 | 0.0036 |
| - Test criteria | | | | |
| Sodium taurocholate: 2.0% or less | | | | |
| Sodium taurochenodeoxycholate: 1.0<> or less | | | | |
| Total related substances: 3.0% or less | | | | |
| Unknown related substances: 0.10 or less | | | | |

**[Table 5]**

| Item | Amount of specimen taken (mg) | Peak area | Related substances (%) | Average (%) |
|---|---|---|---|---|
| Taurine | 100.01 | N/D | Below detection limit | Below detection limit |
| | 100.14 | N/D | Below detection limit | |
| | 100.02 | N/D | Below detection limit | |
| - Test criteria: 2.0% or less | | | | |

**[Table 6]**

| Item | Before drying | | After drying | Loss on drying (%) |
|---|---|---|---|---|
| | Weighing bottle weight (g)-A | Weighing bottle weight + specimen weight (g)-B | Weighing bottle + specimen weight (g)-C | |
| 1 | 27.84474 | 28.84459 | 28.82424 | 2.035 |
| 2 | 25.00061 | 26.00809 | 25.99304 | 1.493 |
| 3 | 27.19929 | 28.19937 | 28.17 | 2.005 |
| Result value | | | 1.84 | |

**[Table 7]**

| Item | Result |
|---|---|
| Heavy metals | 20 ppm or less |
| - Test criteria: 20 ppm or less | |

**[Table 8]**

| Item | Residual solvent amount (ppm) | | | |
|---|---|---|---|---|
| | Test solution 1 | Test solution 2 | Test solution 3 | Average |
| Methanol | 13.9 | 11.3 | 10.4 | 12.0 |
| Ethanol | 6.1 | 5.2 | 5.8 | 5.7 |
| Ethyl acetate | 689.4 | 647.3 | 663.6 | 666.7 |
| Acetone | 12.7 | 12.2 | 12.3 | 12.4 |
| 2-Propanol | 0.2 | 2.4 | 2.7 | 1.7 |
| - Test criteria | | | | |
| Methanol: 3000 ppm or less | | | | |
| Ethanol: 50000 ppm or less | | | | |
| Ethyl acetate: 5000 ppm or less | | | | |
| Acetone: 5000 ppm or less | | | | |
| 2-Propanol: 5000 ppm or less | | | | |

As shown in Tables 3 to 8, it has been confirmed that sodium taurodeoxycholate synthesized on a large scale including the purification method of the present invention has a significantly high purity as the contents of sodium deoxycholate, related substances, heavy metals and the like are significantly low.

### <Experimental Example 3-2> Purity test

Sodium taurodeoxycholate purified according to <Example 2> to <Example 5> was sampled at each step, and the purity thereof was analyzed by the same method as above. The analysis results are as shown in Table 9.

**[Table 9]**

| Item | RRT and area° of peak | | | | Yield |
|---|---|---|---|---|---|
| | Unknown 1 (%) | Unknown 2 (%) | Isomer (%) | Product (wet) (%) | |
| RRT | 0.44 | 0.69 | 0.85 | 1.0 | |
| Crude TDC | 0.073 | 2.754 | 0.457 | 96.715 | 73.00 |
| First recrystallization | 0.044 | 1.000 | 0.453 | 98.503 | 54.75 |
| Second recrystallization | 0.038 | 0.289 | 0.449 | 99.223 | 41.06 |
| Third recrystallization | 0.075 | 0.162 | 0.339 | 99.364 | 30.80 |
| Fourth recrystallization | 0.038 | 0.086 | 0.349 | 99.527 | 26.18 |
| Fourth & Dry | 0.040 | 0.057 | 0.345 | 99.558 | 25.00 |

As shown in Table 9, it has been confirmed that the purity is significantly high when sodium taurodeoxycholate is prepared on a large scale including the purification (recrystallization) method of the present invention. Specifically, <Example 3-1>, <Example 3-2>, <Example 4> and <Example 5> correspond to one time of purification (recrystallization), respectively. When the number of recrystallization is 2 or more times, the purity corresponds to 99% or more and the yield is also as high as 25% or more, so it has been confirmed that sodium taurodeoxycholate can be prepared on a large scale.

### <Comparative Example> Synthesis of sodium taurodeoxycholate by other synthesis methods

### <1-1> Synthesis using isobutyl chloroformate

Sodium taurodeoxycholate was synthesized through the same procedure as in <Example 2>. Sodium taurate, deoxycholic acid (DCA), and isobutyl chloroformate were charged into a reactor, and sodium taurodeoxycholate was synthesized while changing the temperature, solvent, and base. The synthesis procedure was carried out by the following chemical reactions.

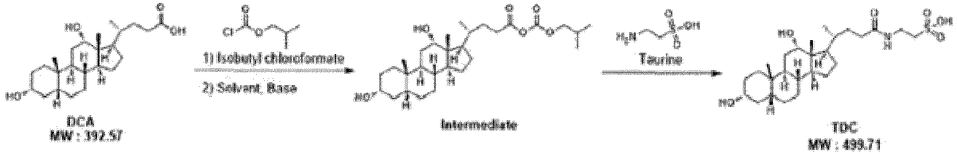

In the synthesis method, the first step reaction was poor, and the reaction conditions in which DCA was quantitatively converted were found by changing the temperature and/or solvent, but thereafter, the reaction rate was low in the second step, so the yield was remarkably low even when the reaction was conducted while changing the solvent, temperature, and base.

### <1-2> Synthesis using pivaloyl chloride

Sodium taurodeoxycholate was synthesized through the same procedure as in <Example 2>. Sodium taurate, deoxycholic acid (DCA), and pivaloyl chloride were charged into a reactor, and sodium taurodeoxycholate was synthesized while changing the temperature and/or solvent. The synthesis procedure was carried out by the following chemical reactions.

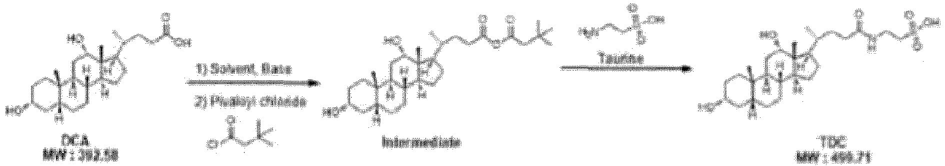

In the synthesis method, DCA, the starting material, was not completely converted in the first step, and the conversion to TDC was less than 50% in the second step as well. Therefore, it was confirmed that the reaction rate was low even the reaction solvent, reaction temperature, and base were changed.

### <1-3> Synthesis using TPP/DTBT

Sodium taurodeoxycholate was synthesized through the same procedure as in <Example 2>. Sodium taurate, deoxycholic acid, triphenylphosphine (TPP) and DTBT were charged into a reactor, and sodium taurodeoxycholate was synthesized while changing the temperature, solvent, and base. The synthesis procedure was carried out by the following chemical reactions.

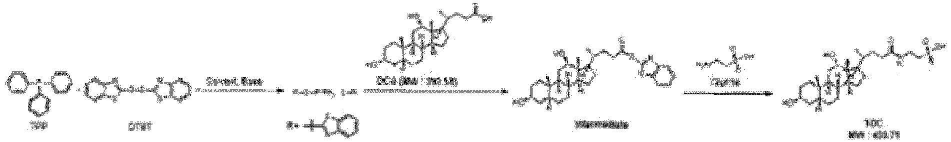

In the synthesis method, the reaction for forming the activating group proceeded quickly in the first step, but the reaction with DCA did not completely proceed after that. In addition, there was a problem that the starting material DCA and TPP/DTFT salt remained after the reactions and it was difficult to completely remove these since the solubility thereof was similar, and it was confirmed that the reaction rate in the second step decreased to 20% to 30%.

### <1-4> Synthesis using DCC/HOBt/MMP

Sodium taurodeoxycholate was synthesized through the same procedure as in <Example 2>. Sodium taurate, deoxycholic acid, DCC (N,N'-dicyclohexylcarbodiimide), HOBt (hydroxybenzotriazole), and MMP were charged into a reactor, and sodium taurodeoxycholate was synthesized while changing the temperature and solvent. The synthesis procedure was carried out by the following chemical reactions.

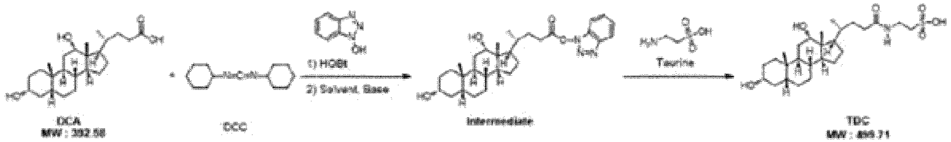

In the synthesis method, complete conversion to an active ester was achieved in the first step, and the active ester of the starting material was completely lost in the second step reaction. Although the reactivity was high, impurities were generated at a high content. In addition, it was confirmed that the work-up procedure was complicated and impurities were not removed smoothly, so that the yield and purity were as low as 65% to 70% and 70% to 75%, respectively. Specific purity is as shown in Table 10 below.

**[Table 10]**

| RT | Imurity Area (%) | Removable or not | Solvent for recrystallization |
|---|---|---|---|
| 3.2 min | 17.75% | Unremovable | EA washing (11 times) |
| 4.8 min | 2-3% | Removable | Recrystallization from IPA:H₂O (4 times) |

### <1-5> Synthesis using DSC

Sodium taurodeoxycholate was synthesized through the same procedure as in <Example 2>. Sodium taurate, deoxycholic acid, and DSC (N,N'-disuccinimudyl carbonate) were charged into a reactor, and sodium taurodeoxycholate was synthesized while changing the temperature and solvent. The synthesis procedure was carried out by the following chemical reactions.

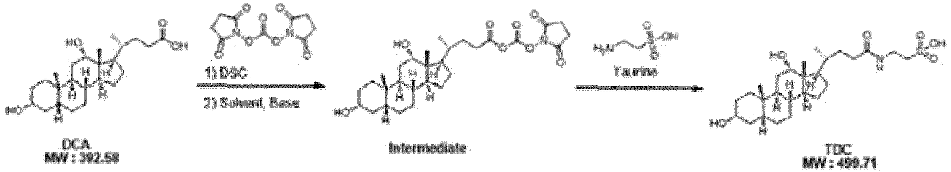

In the synthesis method, on TLC, complete conversion to an active ester was achieved in the first step, but a large amount of impurities were generated in the second step. Although the reaction proceeds easily at a low temperature, it is difficult to remove new impurities and impurities, and the yield and purity have been confirmed to be as low as 55% to 60% and 0.2%, respectively. Specific purity is as shown in Table 11 below.

**[Table 11]**

| RT | Imurity Area (%) | Removable or not | Solvent for recrystallization |
|---|---|---|---|
| 2.5 min | 64.21 | Unremovable | Washing with organic solvent, recrystallization from IPA:H₂O |
| 2.8 min | 26.26% | Unremovable | Washing with organic solvent, recrystallization from IPA:H₂O |
| 4.8 min | - | - | Too small amount to be detected |
| 8.6 min | 0.2% | Unremovable | Washing with organic solvent, recrystallization from IPA:H₂O |

### <1-6> Synthesis using HATU

Sodium taurodeoxycholate was synthesized through the same procedure as in <Example 2>. Sodium taurate, deoxycholic acid, and HATU (1-[bis(dimethylamino)methylene]-1H-1,2,3-triazolo[4,5-b]pyridinium 3-oxide hexafluorophosphate) were charged into a reactor, and sodium taurodeoxycholate was synthesized while changing the temperature and solvent. The synthesis procedure was carried out by the following chemical reactions.

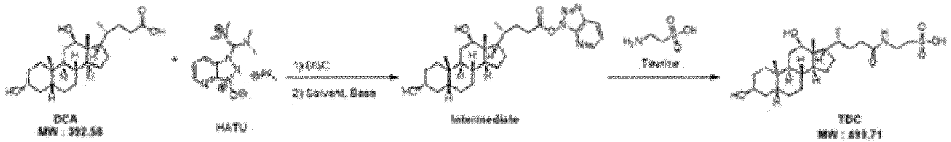

In the synthesis method, complete conversion to an active ester was achieved in the first step, the active ester was completely converted in the second step as well, and thus the reactivity was greatly excellent. However, in the case of related substances, new impurities were also formed together with TDC, and it was confirmed that it was relatively difficult to remove by-products. Impurities and by-products were required to be removed by being dissolved in H₂O, but TDC also dissolved well in H₂O, it was thus confirmed that it was difficult to separate impurities and by-products from TDC.

### [Industrial Applicability]

The present invention can be usefully utilized in industrial fields such as chemistry, medicine, and pharmaceuticals as it has been revealed that the mass production of sodium taurodeoxycholate is possible.

## Claims

1. A mass production method of sodium taurodeoxycholate, comprising:
1) a step of synthesizing crude sodium taurodeoxycholate;
2) a step of washing the crude sodium taurodeoxycholate synthesized in step 1) using an organic solvent and performing filtration to obtain a cake; and
3) a purification step of mixing the cake obtained in step 2) with a solution containing isopropyl alcohol, then i) stirring the mixture with heating for dissolution, ii) stirring the solution with cooling for recrystallization, and iii) washing the recrystallized product with isopropyl alcohol and performing filtration.

2. The mass production method of sodium taurodeoxycholate according to claim 1,
wherein the crude sodium taurodeoxycholate in step 1) is synthesized by placing a solution containing sodium taurate, deoxycholic acid, and N-ethoxycarbonyl-2-ethoxy-1,2-dihydroquinoline (EEDQ) in a stirrer and stirring the solution while adjusting a temperature.

3. The mass production method of sodium taurodeoxycholate according to claim 1,
wherein the organic solvent in step 2) is one or more selected from the group consisting of ethanol, acetone, pyridine, hexafluoroisopropanol, propanol, butanol, cyclohexane, toluene, dichloromethane, diethyl ether, ethyl acetate, methyl acetate, and a mixed solvent of two or more thereof.

4. The mass production method of sodium taurodeoxycholate according to claim 1,
wherein the organic solvent in step 2) is a mixed solvent containing ethanol and acetone, wherein a mixed volume ratio of ethanol to acetone is 1:0.5 to 2.

5. The mass production method of sodium taurodeoxycholate according to claim 1,
wherein step 3) is repeated two or more times.

6. The mass production method of sodium taurodeoxycholate according to claim 1,
wherein step 3) is repeated two to three times.

7. The mass production method of sodium taurodeoxycholate according to claim 1,
the solution containing isopropyl alcohol in step 3) contains water and isopropyl alcohol, wherein a mixed volume ratio of water to isopropyl alcohol is 1:1 to 10.

8. The mass production method of sodium taurodeoxycholate according to claim 1,
wherein the heating in i) of step 3) is heating at 20°C to 100°C.

9. The mass production method of sodium taurodeoxycholate according to claim 1,
wherein the cooling in ii) of step 3) is cooling at 0°C to 50°C.

10. The mass production method of sodium taurodeoxycholate according to claim 1,
wherein the stirring in ii) of step 3) is stirring for 8 to 30 hours.

11. The mass production method of sodium taurodeoxycholate according to claim 1,
wherein isopropyl alcohol in the solution containing isopropyl alcohol in step 3) is 5 to 20 times the weight of the cake in step 2).

12. The mass production method of sodium taurodeoxycholate according to claim 1, further comprising:
4) a step of purifying sodium taurodeoxycholate obtained in step 3) using a mixed solution containing acetone.

13. The mass production method of sodium taurodeoxycholate according to claim 12,
wherein step 4) includes:
A) a step of dissolving sodium taurodeoxycholate filtered in step 3) in a mixed solution containing acetone;
B) a step of adding acetone dropwise to the solution prepared in step A), and recrystallizing sodium taurodeoxycholate while performing cooling and stirring; and
C) a step of washing the recrystallized sodium taurodeoxycholate with acetone and performing filtration and drying.

14. The mass production method of sodium taurodeoxycholate according to claim 1,
wherein it is possible to manufacture 1 kg or more at one time of production by the mass production method of sodium taurodeoxycholate.

15. Sodium taurodeoxycholate produced according to any one of claims 1 to 14.
